# EUROPEAN PATENT APPLICATION

(11) **EP 4 663 657 A1**
(43) Date of publication of application: **17.12.2025**
(21) Application number: 24182313.7
(22) Date of filing: 14.06.2024
(51) Int. Cl.: C07K 16/24, C07K 16/28

(54) **IL-3 SIGNALING INHIBITOR FOR USE IN A METHOD OF TREATMENT OF LONG-COVID AND/OR POST-COVID SYNDROME**

(71) Applicant: Universität Regensburg, 93053 Regensburg (DE); Universitätsklinikum Regensburg, 93053 Regensburg (DE)
(72) Inventor: The designation of the inventor has not yet been filed
(74) Representative: Engelhard, Markus

(57) **Abstract**

The present invention relates to novel means for the treatment of Long-COVID and/or Post-COVID syndrome in patients after infection with a coronavirus. In particular, the present invention relates to an IL-3 signaling inhibitor for use in the treatment of Long-COVID and/or Post-COVID syndrome in a patient. Moreover, the present invention relates to a pharmaceutical composition comprising an IL-3 signaling inhibitor for use in the treatment of Long-COVID and/or Post-COVID syndrome in a patient.

## Description

### FIELD OF THE INVENTION

The present invention relates to the field of the treatment of Long-COVID and/or Post-COVID syndrome. In particular, the present invention relates to an IL-3 signaling inhibitor for use in a method of treatment of Long-COVID and/or Post-COVID syndrome in a patient.

### BACKGROUND OF THE INVENTION

Soon after emergence of the COVID-19 epidemic, it became clear that a considerable proportion of COVID-19 patients suffers from a variety of long-term sequelae of the SARS-CoV-2 infection. More than 200 different symptoms were described including fatigue, muscle weakness, dyspnea, and neuro-psychological symptoms. According to the British National Institute for Health and Care Excellence, Long-COVID is defined as the continuation or development of disease symptoms 4 weeks or more after the start of acute SARS-CoV-2 infection. The definition of Long-COVID also encompasses the Post-COVID syndrome, which is defined by the WHO as the continuation or development of disease symptoms 3 months after the initial SARS-CoV-2 infection, with these symptoms lasting for at least 2 months with no other explanation.

While some meta-analyses reported a quite high incidence of individuals developing Long-COVID symptoms after infection, more controlled studies revealed a frequency of Long-COVID in the range of 5 % of infected adults. More recent virus variants may be associated with a lower risk for Long-COVID. Risk factors for development of Long-COVID are female sex, age, comorbidities, the severity of acute disease, and obesity. While Long-COVID is considered as disease entity with large clinical and socioeconomic impact, the pathophysiology of Long-COVID is poorly understood and no targeted treatments are available. Several potential disease mechanisms were described in patients with Long-COVID including autonomic nervous system damage, complement dysregulation, autoimmunity, endothelial dysfunction, occult viral persistence and thromboinflammation. Long-COVID shows some similarity with the rare chronic fatigue syndrome (ME/CFS) associated with infections and autoimmunity and with the fatigue frequently seen in patients with Sjogren's syndrome and rheumatoid arthritis, suggesting that altered immune reactions could also contribute to development of Long-COVID or Post-COVID sndrome.

Previous studies described pronounced alterations of immune cell function and phenotype in patients with acute SARS-CoV-2 infection, which was associated with a prominent hypo-reactivity of T cells during acute severe SARS-CoV-2 infections. However, immunophenotyping studies with a focus on T cells in clinically well-characterized Long-COVID patients are limited. Hence, it is currently unknown whether the immunological changes during acute infection would persist in patients with Long-COVID, or whether Long-COVID would be associated with different immunological changes.

Therefore, there is a need for further studies that elucidate immunological changes that are associated with Long-COVID and/or Post-COVID syndrome. Moreover, there is a need for treatment options of Long-COVID and/or Post-COVID syndrome.

### SUMMARY OF THE INVENTION

It is therefore an object of the present invention to provide treatment options for Long-COVID and/or Post-COVID syndrome. The object of the present invention is solved by an IL3 signaling inhibitor for use in a method of treatment of Long-COVID and/or Post-COVID syndrome. Moreover, it is an object of the present invention that this IL-3 signaling inhibitor should preferably prevent the activation of the IL-3 receptor.

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to an IL-3 signaling inhibitor for use in a method of treatment of Long-COVID and/or Post-COVID syndrome after infection with a coronavirus in a patient in need thereof, wherein, preferably, the coronavirus is SARS-CoV-2 or a variant thereof.

In one embodiment, the patient is a human or an animal, preferably a human.

In one embodiment, said Long-COVID and/or Post-COVID syndrome is accompanied by an increased IL-3 expression compared to a healthy state.

In one embodiment, the IL-3 signaling inhibitor is selected from a group comprising antibodies including bispecific antibodies and nanobodies, fragments of said antibodies capable of binding an antigen, fusion proteins, antibody prodrugs, oligopeptides, small molecule inhibitors, oligonucleotides, such as aptamers, siRNAs, and shRNAs, and nucleic acids such as DNA and RNA, encoding said antibodies including bispecific antibodies and nanobodies, fragments of said antibodies capable of binding an antigen, fusion proteins, antibody prodrugs, oligopeptides, and oligonucleotides, such as aptamers, siRNAs, and shRNAs;
wherein, preferably, said IL-3 signaling inhibitor is an antibody or a fragment thereof capable of binding an antigen, an oligopeptide, or a small molecule inhibitor; wherein, more preferably, said IL-3 signaling inhibitor is an antibody or a fragment thereof capable of binding an antigen.

In one embodiment, the IL-3 signaling inhibitor is an inhibitor that targets IL-3, an inhibitor that targets interleukin-3 receptor (IL-3R), and/or an inhibitor that targets a complex formed by IL-3 and IL-3R;
wherein, preferably, said IL-3 signaling inhibitor is an anti-IL-3 antibody or an IL-3 binding fragment thereof, or an anti-IL-3R antibody or an IL-3R binding fragment thereof; more preferably an anti-IL-3 antibody or an IL-3 binding fragment thereof.

In a preferred embodiment, the IL-3 is human IL-3, the IL-3R is human IL-3R, and the complex formed by IL-3 and IL-3R is a complex formed by human IL-3 and IL-3R.

In one embodiment, said IL-3 signaling inhibitor is an anti-IL-3 antibody or an IL-3 binding fragment thereof that is capable of preventing IL-3 from binding to and/or activating the IL-3R upon binding of said anti-IL-3 antibody or said IL-3 binding fragment thereof to IL-3 in said patient; or
wherein said IL-3 signaling inhibitor is an anti-IL-3R antibody or an IL-3R binding fragment thereof that is capable of preventing IL-3 from binding to and/or activating the IL-3R upon binding of said anti-IL-3R antibody or an IL-3R binding fragment thereof to IL-3R in said patient.

In one embodiment, said IL-3 signaling inhibitor is an anti-IL-3 antibody or an IL-3 binding fragment thereof, wherein said anti-IL-3 antibody or IL-3 binding fragment thereof binds to the human IL-3 protein.

In one embodiment, said anti-IL-3 antibody or IL-3 binding fragment thereof is not immunogenic in a human subject.

In one embodiment, said anti-IL-3R antibody or IL-3R binding fragment thereof is not immunogenic in a human subject.

In one embodiment, said anti-IL-3 antibody comprises an amino acid sequence which is at least 90%, preferably at least 95%, more preferably at least 98%, more preferably at least 99% identical to the amino acid sequence of an antibody selected from the group comprising:
- monoclonal anti-IL-3 antibody GRT002-H16L2, as described in WO 2024/033522 and international patent application PCT/EP2023/072293 (filing date: August 11, 2023)
- monoclonal anti-IL-3 antibody Clone P8C11C8-6, as described in WO 2017/081218 and international patent application PCT/EP2016/077371 (filing date: November 10, 2023)
- monoclonal anti-IL-3 antibody Clone 13.4.4, as described in European Patent Application No. EP2855524 B1 and international patent application PCT/EP2013/061121 (filing date: May 29, 2013);
preferably of an antibody from the group comprising:
- monoclonal anti-IL-3 antibody GRT002-H16L2
- monoclonal anti-IL-3 antibody Clone P8C11C8-6;
more preferably, of the monoclonal anti-IL-3 antibody GRT002-H16L2.

In one embodiment, said IL-3 binding fragment of said anti-IL-3 antibody comprises a fragment of an anti-IL-3 antibody which comprises an amino acid sequence which is at least 90%, preferably at least 95%, more preferably at least 98%, more preferably at least 99% identical to the amino acid sequence of an antibody selected from the group comprising:
- monoclonal anti-IL-3 antibody GRT002-H16L2
- monoclonal anti-IL-3 antibody Clone P8C11C8-6
- monoclonal anti-IL-3 antibody Clone 13.4.4;
preferably of an antibody from the group comprising:
- monoclonal anti-IL-3 antibody GRT002-H16L2
- monoclonal anti-IL-3 antibody Clone P8C11C8-6;
more preferably, of the monoclonal anti-IL-3 antibody GRT002-H16L2.

In one embodiment, said IL-3 signaling inhibitor is an anti-IL-3 antibody or an IL-3 binding fragment thereof, wherein said anti-IL-3 antibody or said IL-3 binding fragment thereof binds to an epitope within an amino acid sequence which is at least 90% identical, preferably at least 95% identical, more preferably at least 98% identical, even more preferably at least 99% identical, optionally identical, to the mature human IL-3 amino acid sequence (without signal peptide) (SEQ ID NO: 1);
wherein, preferably, said anti-IL-3 antibody or said IL-3 binding fragment thereof binds to a portion of an IL-3 amino acid sequence which is at least 90% identical, preferably at least 95% identical, more preferably at least 98% identical, even more preferably at least 99% identical, optionally identical, to residues 22-48 of the mature human IL-3 amino acid sequence (numbering without signal peptide) (SEQ ID NO: 2).

In one embodiment, said IL-3 signaling inhibitor is a monoclonal, polyclonal, or chimeric antibody, or a combination thereof.

In one embodiment, the patient is characterized as a patient with a Long-COVID score of 1, 2, 3, or 4; preferably, a Long-COVID score of 2, 3, or 4; more preferably a Long-COVID score of 2 or 3; most preferably a Long-COVID score of 3.

A second aspect relates to a pharmaceutical composition comprising an IL-3 signaling inhibitor as defined herein, for use in a method of treatment of Long-COVID and/or Post-COVID syndrome after infection with a coronavirus, wherein, preferably, said composition further comprises at least one pharmaceutically acceptable carrier, diluent and/or excipient.

In one embodiment, said IL-3 signaling inhibitor for use or said pharmaceutical composition for use is administered to a patient in need thereof, wherein, preferably, said patient is a human.

In one embodiment, said IL-3 signaling inhibitor or said pharmaceutical composition is administered in combination with one or more other therapies.

Another aspect of the present application relates to a method of treating a patient with Long-COVID and/or Post-COVID syndrome after infection with a coronavirus, wherein said method comprises administering an IL-3 signaling inhibitor and/or a pharmaceutical composition comprising said IL-3 signaling inhibitor.

According to this aspect, the IL-3 signaling inhibitor, the pharmaceutical composition, the patient, and the coronavirus are as defined herein.

Another aspect of the present application relates to the use of an IL-3 signaling inhibitor and/or a pharmaceutical composition comprising said IL-3 signaling inhibitor for the manufacture of a medicament for treatment of Long-COVID and/or Post-COVID syndrome after infection with a coronavirus in a patient.

According to this aspect, the IL-3 signaling inhibitor, the pharmaceutical composition, the patient, and the coronavirus are as defined herein.

Another aspect of the present application relates to a medicament comprising an IL-3 signaling inhibitor and/or a pharmaceutical composition comprising said IL-3 signaling inhibitor for use in the treatment of Long-COVID and/or Post-COVID syndrome after infection with a coronavirus in a patient.

According to this aspect, the IL-3 signaling inhibitor, the pharmaceutical composition, the patient, and the coronavirus are as defined herein.

### DETAILED DESCRIPTION

In one embodiment, the term "patient" relates to a human or an animal, preferably a human. The terms "of the [present] invention", "in accordance with the invention", "according to the invention" and the like, as used herein, are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B, and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

The term "Post-COVID syndrome", as used herein, refers to the continuation or development of disease symptoms 3 months after the initial SARS-CoV-2 infection, with these symptoms lasting for at least 2 months with no other explanation. Typical symptoms may include fatigue, shortness of breath, cognitive dysfunction but also others, and generally have an impact on everyday functioning. Symptoms may be new onset following initial recovery from an acute COVID-19 episode or persist from the initial illness. Symptoms may also fluctuate or relapse over time. In the International classification of Diseases by the WHO, ICD-11, such condition is codified by code RA02. Such code can be currently (May 2024) found under the following link: https://icd.who.int/browse/2024-01/mms/en*2024855916. Post-COVID syndrome may also be referred to as "Post-COVID condition", "Post-COVID-19 syndrome" and the like.

The term "Long-COVID", as used herein, refers to the continuation or development of disease symptoms 4 weeks or more after the start of acute SARS-CoV-2 infection. Typical symptoms may include fatigue, shortness of breath, cognitive dysfunction but also others, and generally have an impact on everyday functioning. Symptoms may be new onset following initial recovery from an acute COVID-19 episode or persist from the initial illness. Symptoms may also fluctuate or relapse over time. By this, the term "Long-COVID" also encompasses the "Post-COVID syndrome".

The term "Long-COVID score", as used herein, is based on a score described by Klok *et al.*, 2020¹. The Long-COVID score may also be referred to as "Long-COVID severity score". Using the Long-COVID score, a Long-COVID cohort is classified based on self-assessment of the severity of their illness as follows: 0 = no limitations in everyday life and no symptoms, pain, depression or anxiety related to the infection, 1 = negligible limitations in everyday life, performance of all usual duties/activities, however with persistent symptoms, pain, depression or anxiety related to the infection, 2 = limitations in everyday life with occasionally need to avoid or reduce usual duties/activities or to spread these over time due to symptoms, pain, depression or anxiety related to the infection. Patients are able to perform all activities without any assistance. 3 = limitations in everyday life with inability to perform all usual duties/activities due to symptoms, pain, depression or anxiety related to the infection. Patients are able to take care of themselves without any assistance. 4 = severe limitations in everyday life with inability to take care of themselves and dependence on nursing care and/or assistance from other persons due to symptoms, pain, depression or anxiety related to the infection.

The term "SARS-CoV-2", as used herein, refers to "Severe acute respiratory syndrome coronavirus 2", a strain of coronavirus that causes COVID-19, the respiratory illness responsible for the COVID-19 pandemic. The virus is sometimes also referred to as "(human) coronavirus 19", "HCov-19". SARS-CoV-2 is a strain of the species severe-acute-respiratory-syndrome-related coronavirus (SARSr-CoV). The term "variant of SARS-CoV-2" or the like, as used herein, refers to SARS-CoV-2 variants that harbor one or several mutations in the genome of the virus. Variants of SARS-CoV-2 may have one or several polymorphic nucleotide positions in various reading frames of the viral genome (e.g. spike protein ORF, ORF1a, ORF1b, ORF3A, ORF8 and nucleocapsid protein ORF). SARS-CoV-2 variants include, however are not limited to, Delta variant, Omicron variant, Alpha variant, Gamma variant, Beta variant, as well as subvariants and different lineages thereof.

The term "IL-3 signaling inhibitor", as used herein, refers to a molecule that inhibits the signaling cascade that is initiated by the interaction of IL-3 with the IL-3R. Preferably, said IL-3 signaling inhibitor is capable of preventing IL-3 from binding to and/or activating the IL-3R. This relates to a situation where, upon interaction of an IL-3 signaling inhibitor with an IL-3 protein molecule or an IL-3R protein molecule, said IL-3 protein molecule is not capable of inducing activation of IL-3R anymore. "Activation" of the IL-3 receptor denotes the molecular processes which an IL-3 receptor undergoes upon binding of IL-3 that result in transduction of a signal to the interior of an IL-3 receptor-bearing cell to bring about changes in cellular physiology. Such changes in cellular physiology in response to IL-3 receptor activation are typically activation of the JAK-STAT pathway, the Ras-MAP kinase pathway, and the PI-3 kinase pathway. IL-3 is known to activate e.g. basophils, monocytes, dendritic cells, B cells, T cells, endothelial cells.

As used herein, the terms "IL-3" or "interleukin-3" are synonymous and refer to the naturally occurring, or endogenous mammalian interleukin-3 proteins and to proteins having an amino acid sequence which is the same as that of a naturally occurring or endogenous corresponding mammalian IL-3 protein (e.g. recombinant proteins, synthetic proteins (i.e., produced using the methods of synthetic organic chemistry)). Such proteins can for example be recovered or isolated from a source which naturally produces IL-3, or be produced by methods of recombinant protein expression. The terms "IL-3" or "interleukin-3" comprise the IL-3 proteins of different mammalian organisms, such as human, mouse, or rat IL-3. In one embodiment, the terms relate exclusively to human IL-3. Preferably, the amino acid sequence of human IL-3 is provided by SEQ ID NO: 1.

As used herein, the terms "IL-3R", "IL-3 receptor", or "interleukin-3 receptor" are synonymous and refer to the naturally occurring or endogenous mammalian interleukin 3 receptor proteins and to proteins having an amino acid sequence which is the same as that of a naturally occurring or endogenous corresponding mammalian IL-3R protein (e.g. recombinant proteins, synthetic proteins (i.e., produced using the methods of synthetic organic chemistry)). Such proteins can for example be recovered or isolated from a source which naturally produces IL-3r or be produced by methods of recombinant protein expression. The terms "IL-3R", "IL-3 receptor", or "interleukin-3 receptor" comprise the IL-3R proteins of different mammalian organisms, such as human, mouse, or rat IL-3. In a preferred embodiment, the terms relate to human IL-3R. The IL-3R may also be referred to as "CD123".

At some instances, the present application refers to a certain disease being characterized by an "increased IL-3 expression compared to a healthy state". Preferably, this term refers to an increase in the number of IL-3 expressing CD8⁺ T cells compared to a healthy state. Such an increased IL-3 expression compared to a healthy state can be determined according to methods known in the art. For example, the number of IL-3 expressing CD8⁺ T cells compared to a healthy state can be determined by analyzing the total number of IL-3 expressing CD8⁺ T cells in a blood sample of a patient with Long-COVID or Post-COVID syndrome, e.g. by intracellular cytokine staining of T cells, and comparing the value obtained with typical values as obtained by the same assay with samples from healthy individuals (i.e. individuals that are free of any disease that may affect IL-3 expression, preferably free of any disease).

The term "antibody", as used herein, is used interchangeably with the term "immunoglobulin" and refers to a glycoprotein comprising at least two heavy (H) chains and two light (L) chains inter-connected by disulfide bonds. The term also includes all recombinant forms of antibodies, e.g. antibodies expressed in prokaryotes, unglycosylated antibodies and derivatives as described below. There are five different types of heavy chains, which define antibody isotypes of different functional activity: IgM, IgD, IgG, IgA and IgE. Each heavy chain comprises a heavy chain variable region (abbreviated herein as V_{H} region) and a heavy chain constant region. Each light chain comprises a light chain variable region (abbreviated herein as V_{L} region) and a light chain constant region. The V_{H} and V_{L} regions can be further subdivided into regions of hypervariability, termed complementarity determining regions (CDR), interspersed with regions that are more conserved, termed framework regions (FR). The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The terms "fragment of an antibody", "binding fragment of an antibody", "antibody fragment" and the like, as used herein, refer to one or more portions of an antibody that retain the ability to specifically interact (e.g. by binding, steric hindrance, stabilizing spatial distribution) an antigen. Examples of binding fragments include, but are not limited to, a Fab fragment, a monovalent fragment consisting of the V_{L}, V_{H}, CL and CH1 domains; a F(ab)2 fragment, a bivalent fragment comprising two Fab fragments linked by a disulfide bridge at the hinge region; a Fd fragment consisting of the V_{H} and CHI domains; a Fv fragment consisting of the V_{L} and V_{H} domains of a single arm of an antibody; a dAb fragment, which consists of a V_{H} domain; and an isolated complementarity determining region (CDR). Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv)). Such single chain antibodies are also intended to be encompassed within the term "fragment of an antibody", "binding fragment of an antibody", "antibody fragment" and the like.

The term "monoclonal antibody", as used herein, refers to a preparation of antibody molecules of single molecular composition. A monoclonal antibody displays a single binding specificity and affinity for a particular epitope. In one embodiment, the monoclonal antibodies are produced by a hybridoma which includes a B cell obtained from a non-human animal, e.g. mouse, fused to an immortalized cell.

As used herein, the term "polyclonal antibody" refers to a heterogeneous pool of antibodies produced by a number of different B lymphocytes. Different antibodies in the pool recognize and specifically bind different epitopes.

The term "chimeric antibody" refers to those antibodies wherein one portion of each of the amino acid sequences of heavy and light chains is based upon a sequence in an antibody/sequences in antibodies derived from a particular species or belonging to a particular class, while the remaining segment of the chain is based upon a sequence/sequences in another. Typically, the variable region of both light and heavy chains mimics the variable regions of antibodies derived from one species of mammals, while the constant portions are based upon sequences of antibodies derived from another. Preferably, the constant portions are based upon sequences of antibodies derived from human.

The term "humanized antibody", as used herein, refers to a molecule having an antigen binding site that is substantially derived from an immunoglobulin from a non-human species, wherein the remaining immunoglobulin structure of the molecule is based upon the structure and/or sequence of a human immunoglobulin. The antigen binding site may either comprise complete variable domains fused onto constant domains or only the complementarity determining regions (CDR) grafted onto appropriate framework regions in the variable domains. Antigen binding sites may be wild-type or modified by one or more amino acid substitutions, e.g. modified to resemble human immunoglobulins more closely. Some forms of humanized antibodies preserve all CDR sequences (for example a humanized mouse antibody which contains all six CDRs from the mouse antibody). Other forms have one or more CDRs which are altered with respect to the original antibody.

The term "human antibody", as used herein, refers to an antibody which comprises only sequences derived from human immunoglobulin sequences.

The term "synthetic antibody", as used herein, relates to an antibody which is generated using recombinant DNA technology (such as by phage display) or DNA synthesis.

The term "anti-IL-3 antibody", as used herein, refers to an antibody which binds to the IL-3 protein. Preferably, said binding is specific binding. If the present application refers to an "IL-3 binding fragment" of an anti-IL-3 antibody, this relates to a fragment of that anti-IL-3 antibody which fragment is capable of binding to the IL-3 protein. Preferably, said binding is specific binding. The term "anti-IL-3 antibody" also includes monoclonal, polyclonal, humanized, human, and synthetic antibodies, single chain, bispecific, and simianized antibodies, as well as aptamers.

The term "anti-IL-3R antibody", as used herein, refers to an antibody which binds to the IL-3R protein. Preferably, said binding is specific binding. If the present application refers to an "IL-3R binding fragment" of an anti-IL-3R antibody, this relates to a fragment of that anti-IL-3R antibody which fragment is capable of binding to the IL-3R protein. Preferably, said binding is specific binding. The term "anti-IL-3R antibody" also includes monoclonal, polyclonal, humanized, human, and synthetic antibodies, single chain, bispecific, and simianized antibodies, as well as aptamers.

The ability of the antibody to bind an antigen such as IL-3 or IL-3R, can be determined using standard binding assays, such as ELISA, Western Blot, or measurement of affinity (K_{D}) by surface plasmon resonance measurements.

Examples of IL-3 binding fragments of an anti-IL-3 antibody or IL-3R binding fragments of an anti-IL-3R antibody include, but are not limited to, separated light and heavy chains, Fab, Fab/c, Fv, Fab', and F(ab')2 fragments, including epitope-binding fragments of any of the antibodies and fragments mentioned above.

As used herein, the term "epitope" means a protein sequence / structure capable of binding to an antibody generated in response to such sequence, wherein the term "binding" herein preferably relates to specific binding.

The term "immunogenic", as used herein, refers to a peptide which, upon being administered to a subject, or taken up by the subject in other ways, elicits an immune response. Such immune response includes at least the generation of antibodies which specifically bind the immunogenic substance (i.e., a humoral response). An immunogenic substance may in addition elicit a cellular immunological response.

The terms "binds" and "binding", as used herein, preferably relate to specific binding. In some embodiments, the terms "binds" is to be understood as "is capable of binding", and "binding" is to be understood as "capable of binding"; accordingly, the term "is specific for" is to be understood as "is capable of specifically binding (to)", the term "specifically binds" is to be understood as "is capable of specifically binding (to)", and "specific binding" is to be understood as "capability of specific binding (to)".

The terms "is specific for", "specifically binds" and "specific binding", as used for example in the context of a molecule A being specific for a molecule B or a molecule A specifically binding to a molecule B or a molecule A showing specific binding for a molecule B, refer to a situation where molecule A binds to molecule B, but does not bind to other unrelated molecules, or with substantially reduced affinities. Such binding can be measured by routine methods, for example by competition ELISA or by measurement of affinity (K_{D}) by surface plasmon resonance measurements. Similarly, a molecule A being specific for an epitope C or a molecule A specifically binding to epitope C or a molecule A showing specific binding for epitope C, refer to a situation where molecule A binds to epitope C, but does not bind to other unrelated epitopes, or with substantially reduced affinities.

The term "treatment", as used herein, refers to the process of providing a subject with a pharmaceutical treatment, e.g., the administration of a drug, such that at least one symptom of the disease is decreased. Treatment of a disease can be improving the disease and/or curing the disease.

The phrase that something/an event occurs "simultaneously with said administration of said IL-3 signaling inhibitor" or the like, is meant to designate that the event occurs (a) at the same time at which said IL-3 signaling inhibitor is applied to the patient, (b) at a time that lies between individual administrations of said antibody/fragment thereof, if administration of said IL-3 signaling inhibitor occurs in several individual administrations, or (c) after administration of said IL-3 signaling inhibitor, while the administered IL-3 signaling inhibitor is still present in the tissue or blood of the patient.

The term "pharmaceutically acceptable carrier, diluent and/or excipient" refers to a non-toxic, inert, solid, semi-solid, or liquid diluent material or formulation auxiliary of any type. "Pharmaceutically acceptable" in this context is meant to designate that said carrier is compatible with the other ingredients of the pharmaceutical composition and not harmful to the patient that the pharmaceutical composition is administered to. Examples of pharmaceutically acceptable carriers include, but are not limited to, water, water-propylene glycol solutions, or aqueous polyethylene glycol solutions.

The term "effective amount", as used herein, refers to an amount that produces a desired treatment effect in a subject. This amount will vary depending upon a variety of factors, including but not limited to the characteristics of the therapeutic compound (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and the route of administration. A person skilled in the art will be able to determine an effective amount through routine experimentation, namely by monitoring a subject's response to administration of a compound and adjusting the dosage accordingly. For additional guidance, see Remington: The Science and Practice of Pharmacy 20th Edition, Gennaro, Ed., Williams & Wilkins Pennsylvania, 2000.

The term "bispecific antibody", as used herein, refers to a type of antibody that can bind to two different antigens at the same time.

The term "nanobody", as used herein, refers to a single-domain antibody (sdAb), which is an antibody fragment consisting of a single monomeric variable antibody domain. Like a whole antibody, it is able to bind selectively to a specific antigen.

The term "fusion proteins", as used herein, refers to chimeric proteins that are created through the joining of two or more genes that originally coded for separate proteins.

The term "antibody prodrugs", as used herein, refers to masked antibodies engineered to be pharmacologically inactive until they are activated by proteases in the diseased tissue microenvironment.

In one embodiment, the IL-3 signaling inhibitor of the present invention is an inhibitor that targets IL-3, an inhibitor that targets interleukin-3 receptor (IL-3R), and/or an inhibitor that targets a complex formed by IL-3 and IL-3R;
wherein, preferably, said IL-3 signaling inhibitor is an anti-IL-3 antibody or an IL-3 binding fragment thereof, or an anti-IL-3R antibody or an IL-3R binding fragment thereof; more preferably an anti-IL-3 antibody or an IL-3 binding fragment thereof.

In one embodiment, said IL-3 signaling inhibitor is an anti-IL-3 antibody or an IL-3 binding fragment thereof, wherein said anti-IL-3 antibody or IL-3 binding fragment thereof binds to the human IL-3 protein;
wherein, preferably, said anti-IL-3 antibody or IL-3 binding fragment thereof is not immunogenic in a human subject.

In one embodiment, said anti-IL-3 antibody comprises an amino acid sequence which is at least 90%, preferably at least 95%, more preferably at least 98%, more preferably at least 99% identical to the amino acid sequence of an antibody selected from the group comprising:
- monoclonal anti-IL-3 antibody GRT002-H16L2 as described in WO 2024/033522 and international patent application PCT/EP2023/072293 (filing date: August 11, 2023)
- monoclonal anti-IL-3 antibody Clone P8C11C8-6 as described in WO 2017/081218 and international patent application PCT/EP2016/077371 (filing date: November 10, 2023)
- monoclonal anti-IL-3 antibody Clone 13.4.4 as described in European Patent Application No. EP2855524 B1 and international patent application PCT/EP2013/061121 (filing date: May 29, 2013);
preferably of an antibody from the group comprising:
- monoclonal anti-IL-3 antibody GRT002-H16L2
- monoclonal anti-IL-3 antibody Clone P8C11C8-6;
more preferably, of the monoclonal anti-IL-3 antibody GRT002-H16L2.

In one embodiment, said IL-3 binding fragment of said anti-IL-3 antibody comprises a fragment of an anti-IL-3 antibody which comprises an amino acid sequence which is at least 90%, preferably at least 95%, more preferably at least 98%, more preferably at least 99% identical to the amino acid sequence of an antibody selected from the group comprising:
- monoclonal anti-IL-3 antibody GRT002-H16L2
- monoclonal anti-IL-3 antibody Clone P8C11C8-6
- monoclonal anti-IL-3 antibody Clone 13.4.4;
preferably of an antibody from the group comprising:
- monoclonal anti-IL-3 antibody GRT002-H16L2
- monoclonal anti-IL-3 antibody Clone P8C11C8-6;
more preferably, of the monoclonal anti-IL-3 antibody GRT002-H16L2.

In one embodiment, the anti-IL-3 antibody is selected from the group comprising:
- monoclonal anti-IL-3 antibody GRT002-H16L2
- monoclonal anti-IL-3 antibody Clone P8C11C8-6
- monoclonal anti-IL-3 antibody Clone 13.4.4;
preferably from the group comprising:
- monoclonal anti-IL-3 antibody GRT002-H16L2
- monoclonal anti-IL-3 antibody Clone P8C11C8-6;
more preferably, the anti-IL-3 antibody is the monoclonal anti-IL-3 antibody GRT002-H16L2; and/or said IL-3 binding fragment thereof is an IL-3 binding fragment of an antibody selected from the group comprising:
- monoclonal anti-IL-3 antibody GRT002-H16L2
- monoclonal anti-IL-3 antibody Clone P8C11C8-6
- monoclonal anti-IL-3 antibody Clone 13.4.4;
preferably of an antibody from the group comprising:
- monoclonal anti-IL-3 antibody GRT002-H16L2
- monoclonal anti-IL-3 antibody Clone P8C11C8-6;
more preferably, of the monoclonal anti-IL-3 antibody GRT002-H16L2.

In one embodiment said anti-IL-3 antibody or said IL-3 binding fragment thereof competes with an antibody selected from the group comprising:
- monoclonal anti-IL-3 antibody GRT002-H16L2
- monoclonal anti-IL-3 antibody Clone P8C11C8-6
- monoclonal anti-IL-3 antibody Clone 13.4.4;
preferably from the group comprising:
- monoclonal anti-IL-3 antibody GRT002-H16L2
- monoclonal anti-IL-3 antibody Clone P8C11C8-6;
more preferably, the anti-IL-3 antibody is the monoclonal anti-IL-3 antibody GRT002-H16L2

In one embodiment, said anti-IL-3 antibody or said IL-3 binding fragment thereof comprises an amino acid sequence that is identical to the amino acid sequence of the V_{H} region of an antibody selected from the group comprising:
- monoclonal anti-IL-3 antibody GRT002-H16L2
- monoclonal anti-IL-3 antibody Clone P8C11C8-6
- monoclonal anti-IL-3 antibody Clone 13.4.4;

preferably of an antibody from the group comprising:
   - monoclonal anti-IL-3 antibody GRT002-H16L2
   - monoclonal anti-IL-3 antibody Clone P8C11C8-6;
more preferably, of the monoclonal anti-IL-3 antibody GRT002-H16L2;
or that has an amino acid sequence at least 90%, preferably at least 95%, more preferably at least 98%, more preferably at least 99% identical to the amino acid sequence of the V_{H} region of an antibody selected from the group comprising:
   - monoclonal anti-IL-3 antibody GRT002-H16L2
   - monoclonal anti-IL-3 antibody Clone P8C11C8-6
   - monoclonal anti-IL-3 antibody Clone 13.4.4;
preferably of an antibody from the group comprising:
   - monoclonal anti-IL-3 antibody GRT002-H16L2
   - monoclonal anti-IL-3 antibody Clone P8C11C8-6;
more preferably, of the monoclonal anti-IL-3 antibody GRT002-H16L2.

In one embodiment, said anti-IL-3 antibody or said IL-3 binding fragment thereof comprises an amino acid sequence that is identical to the amino acid sequence of the V_{L} region of an antibody selected from the group comprising:
- monoclonal anti-IL-3 antibody GRT002-H16L2
- monoclonal anti-IL-3 antibody Clone P8C11C8-6
- monoclonal anti-IL-3 antibody Clone 13.4.4;

preferably of an antibody from the group comprising:
   - monoclonal anti-IL-3 antibody GRT002-H16L2
   - monoclonal anti-IL-3 antibody Clone P8C11C8-6;
more preferably, of the monoclonal anti-IL-3 antibody GRT002-H16L2;
or that has an amino acid sequence at least 90%, preferably at least 95%, more preferably at least 98%, more preferably at least 99% identical to the amino acid sequence of the V_{L} region of an antibody selected from the group comprising:
   - monoclonal anti-IL-3 antibody GRT002-H16L2
   - monoclonal anti-IL-3 antibody Clone P8C11C8-6
   - monoclonal anti-IL-3 antibody Clone 13.4.4;
preferably of an antibody from the group comprising:
   - monoclonal anti-IL-3 antibody GRT002-H16L2
   - monoclonal anti-IL-3 antibody Clone P8C11C8-6;
more preferably, of the monoclonal anti-IL-3 antibody GRT002-H16L2.

In one preferred embodiment, said anti-IL-3 antibody or said IL-3 binding fragment thereof comprises a HCDR1 region that has an amino acid sequence at least 90%, preferably at least 95%, more preferably at least 98%, more preferably at least 99% identical to the amino acid sequence of the HCDR1 region of SEQ ID NO: 4, as defined by Kabat, or the HCDR1 region of SEQ ID NO: 7, as defined by IMGT.

In one preferred embodiment, said anti-IL-3 antibody or said IL-3 binding fragment thereof comprises a HCDR2 region that has an amino acid sequence at least 90%, preferably at least 95%, more preferably at least 98%, more preferably at least 99% identical to the amino acid sequence of the HCDR2 region of SEQ ID NO: 5, as defined by Kabat, or the HCDR2 region of SEQ ID NO: 8, as defined by IMGT.

In one preferred embodiment, said anti-IL-3 antibody or said IL-3 binding fragment thereof comprises a HCDR3 region that has an amino acid sequence at least 90%, preferably at least 95%, more preferably at least 98%, more preferably at least 99% identical to the amino acid sequence of the HCDR3 region of SEQ ID NO: 6, as defined by Kabat, or the HCDR3 region of SEQ ID NO: 9, as defined by IMGT.

In one preferred embodiment, said anti-IL-3 antibody or said IL-3 binding fragment thereof comprises a LCDR1 region that has an amino acid sequence at least 90%, preferably at least 95%, more preferably at least 98%, more preferably at least 99% identical to the amino acid sequence of the LCDR1 region of SEQ ID NO: 11, as defined by Kabat, or the LCDR1 region of SEQ ID NO: 14, as defined by IMGT.

In one preferred embodiment, said anti-IL-3 antibody or said IL-3 binding fragment thereof comprises a LCDR2 region that has an amino acid sequence at least 90%, preferably at least 95%, more preferably at least 98%, more preferably at least 99% identical to the amino acid sequence of the LCDR2 region of SEQ ID NO: 12, as defined by Kabat, or the LCDR2 region of SEQ ID NO: 15, as defined by IMGT.

In one preferred embodiment, said anti-IL-3 antibody or said IL-3 binding fragment thereof comprises a LCDR3 region that has an amino acid sequence at least 90%, preferably at least 95%, more preferably at least 98%, more preferably at least 99% identical to the amino acid sequence of the LCDR3 region of SEQ ID NO: 13, as defined by Kabat, or the LCDR3 region of SEQ ID NO: 16, as defined by IMGT.

The structures and locations of immunoglobulin variable domains, e.g., CDRs, may be defined using well known numbering schemes, e.g., the Kabat numbering scheme, the Chothia numbering scheme, or a combination of Kabat and Chothia (see, e.g. Sequences of Proteins of Immunological Interest, U.S. Department of Health and Human Services (1991), eds. Kabat et al.; Lazikani et al., (1997) J. Mol. Bio. 273:927-948); Kabat et al., (1991) Sequences of Proteins of Immunological Interest, 5th edit., NIH Publication no. 91-3242 U.S. Department of Health and Human Services; Chothia et al., (1987) J. Mol. Biol. 196:901 -917; Chothia et al., (1989) Nature 342:877-883; and Al-Lazikani et al., (1997) J. Mol. Biol. 273:927-948; Annals of the New York Academy of Sciences, 764,47-49 (1995); Nucleic Acids Research, 25, 206-211(1997).

Alternatively, the structures and locations of immunoglobulin variable domains, e.g., CDRs, may be defined using well known IMGT numbering schemes (IMGT^{®} of ImMunoGeneTics information system^{®}).

In one preferred embodiment, said anti-IL-3 antibody or said IL-3 binding fragment thereof comprises a V_{H} region that has an amino acid sequence at least 90%, preferably at least 95%, more preferably at least 98%, more preferably at least 99% identical to the amino acid sequence of SEQ ID NO: 3 or SEQ ID NO: 17, preferably of SEQ ID NO: 17.

In one preferred embodiment, said anti-IL-3 antibody or said IL-3 binding fragment thereof comprises a V_{H} region that has an amino acid sequence at least 90%, preferably at least 95%, more preferably at least 98%, more preferably at least 99% identical to the amino acid sequence of SEQ ID NO: 19.

In one preferred embodiment, said anti-IL-3 antibody or said IL-3 binding fragment thereof comprises a V_{L} region that has an amino acid sequence at least 90%, preferably at least 95%, more preferably at least 98%, more preferably at least 99% identical to the amino acid sequence of SEQ ID NO: 10 or SEQ ID NO: 18, preferably of SEQ ID NO: 18.

In one preferred embodiment, said anti-IL-3 antibody or said IL-3 binding fragment thereof comprises a V_{L} region that has an amino acid sequence at least 90%, preferably at least 95%, more preferably at least 98%, more preferably at least 99% identical to the amino acid sequence of SEQ ID NO: 20.

In one embodiment, said anti-IL-3R antibody comprises an amino acid sequence which is at least 90%, preferably at least 95%, more preferably at least 98%, more preferably at least 99% identical to an amino acid sequence of a Fc-silent variant of the monoclonal anti-IL-3R antibody CSL362 (talacotuzumab).

In one embodiment, said IL-3R binding fragment of said anti-IL-3R antibody comprises a fragment of an amino acid sequence at least 90%, preferably at least 95%, more preferably at least 98%, more preferably at least 99% identical to the amino acid sequence of an amino acid sequence of a Fc-silent variant of the monoclonal anti-IL-3R antibody CSL362 (talacotuzumab).

The term "Fc-silent variant", as used herein, refers to an antibody variant containing genetically engineered Fc domain that contains key point mutations that abrogate binding of Fc receptors, abolishing antibody directed cytotoxicity effector function.

In one embodiment, said anti-IL-3 antibody or said IL-3 binding fragment thereof comprises an amino acid sequence that is identical to the amino acid sequence of the V_{H} region of the monoclonal anti-IL-3R antibody CSL362 (talacotuzumab); or that has an amino acid sequence at least 90%, preferably at least 95%, more preferably at least 98%, more preferably at least 99% identical to the amino acid sequence of the V_{H} region of the monoclonal anti-IL-3R antibody CSL362 (talacotuzumab).

In one embodiment, said anti-IL-3 antibody or said IL-3 binding fragment thereof comprises an amino acid sequence that is identical to the amino acid sequence of the V_{L} region of the monoclonal anti-IL-3R antibody CSL362 (talacotuzumab), or that has an amino acid sequence at least 90%, preferably at least 95%, more preferably at least 98%, more preferably at least 99% identical to the amino acid sequence of the V_{L} region of the monoclonal anti-IL-3R antibody CSL362 (talacotuzumab).

For several antibodies it is known that their binding to human IL-3 prevents IL-3 from binding to its receptor IL-3R, for example monoclonal anti-IL-3 antibody GRT002-H16L2, as described in WO 2024/033522 and international patent application PCT/EP2023/072293 (filing date: August 11, 2023); the monoclonal anti-IL-3 antibody Clone P8C11C8-6, as described in WO 2017/081218 and international patent application PCT/EP2016/077371 (filing date: November 10, 2023); or the monoclonal anti-IL-3 antibody Clone 13.4.4, as described in European Patent Application No. EP2855524 B1 and international patent application PCT/EP2013/061121 (filing date: May 29, 2013).

For several antibodies it is known that their binding to human IL-3R prevents IL-3 from binding to its receptor IL-3R, for example the monoclonal anti-IL-3R antibody CSL362 (talacotuzumab).

In one embodiment, the IL-3 signaling inhibitor, preferably said anti-IL-3 antibody or said IL-3 binding fragment thereof, is administered to a patient in need thereof. Preferably, said patient is a mammal, more preferably said patient is a human being.

In one embodiment, said anti-IL-3 antibody is a monoclonal, polyclonal or chimeric antibody, or a combination thereof. In one embodiment, said IL-3 binding fragment of said anti-IL-3 antibody is a fragment of a monoclonal, polyclonal or chimeric antibody, or a combination thereof.

In one embodiment, said anti-IL-3 antibody or said IL-3 binding fragment thereof is not immunogenic in a human subject.

In one embodiment, said anti-IL-3 antibody is a humanized antibody. In one embodiment, said anti-IL-3 antibody is a human antibody. In one embodiment, said anti-IL-3 antibody is a synthetic antibody.

In one embodiment, said anti-IL-3 antibody is of antibody isotype IgG, IgA, IgM, IgD, or IgE. Preferably, said anti-IL-3 antibody is of antibody isotype IgG.

In one embodiment, said anti-IL-3 antibody is obtained by a method comprising the step of immunizing an animal with a protein or peptide comprising or consisting of the amino acid sequence of SEQ ID NO: 1 or an immunogenic fragment thereof, or a nucleic acid or host cell expressing said protein or peptide or immunogenic fragment thereof, or comprising or consisting of the amino acid sequence of SEQ ID NO: 2 or an immunogenic fragment thereof, or a nucleic acid or host cell expressing said protein or peptide or immunogenic fragment thereof.

Preferably, said fragment of human IL-3 has a length of at least 100, more preferably at least 80, more preferably at least 50, more preferably at least 40, more preferably at least 30, more preferably at least 22, more preferably at least 20, more preferably at least 10 amino acids. Most preferably, said fragment of human IL-3 has a length of at least 10 amino acids.

Preferably, said fragment of human IL-3 has a length of up to 100, more preferably up to 80, more preferably up to 50, more preferably up to 40, more preferably up to 30, more preferably up to 27 amino acids. Most preferably, said fragment of human IL-3 has a length of up to 27 amino acids.

In one embodiment, said anti-IL-3 antibody is obtained by a method comprising the step of immunizing a rabbit, rat, mouse, chicken, goat, guinea pig, hamster, horse, or sheep. In one embodiment, said anti-IL-3 antibody is obtained by a method comprising the step of immunizing a rabbit. In one embodiment, said anti-IL-3 antibody is obtained by a method comprising the step of immunizing a mouse.

In one embodiment, said anti-IL-3 antibody or said IL-3 binding fragment thereof binds to the human IL-3 protein. In one embodiment, said anti-IL-3 antibody or said IL-3 binding fragment thereof is specific for the human IL-3 protein. In one embodiment, said anti-IL-3 antibody or said IL-3 binding fragment thereof does not bind to IL-3 proteins of other species besides human.

In one embodiment, said anti-IL-3 antibody or said IL-3 binding fragment thereof is capable of preventing IL-3 from binding to and/or activating its receptor upon binding of said anti-IL-3 antibody or said IL-3 binding fragment thereof to IL-3. In one embodiment, binding of said anti-IL-3 antibody or said IL-3 binding fragment thereof to IL-3 prevents IL-3 from binding to and/or activating its receptor. Preferably, said receptor is the interleukin-3 receptor. Preferably, said binding or lack of binding of IL-3 to its receptor is determined by enzyme-linked immunosorbent assay (ELISA), more preferably by enzyme-linked immunosorbent assay (ELISA) using recombinant IL-3 receptor alpha-chain, or by determining binding of labelled IL-3, preferably IL-3 labelled with a fluorescent or radioactive label, to IL-3 receptor expressing cells, preferably by immunofluorescence or immunostaining/flow cytometric analysis of cells expressing the IL-3 receptor. Preferably, said binding or lack of binding of IL-3 to its receptor is determined by measurement of affinity (K_{D}) by surface plasmon resonance measurements. Preferably, said binding or lack of binding of IL-3 to its receptor is determined by measurement of affinity (K_{D}) by flow cytometry, cell-based ELISA or detection of bound radioactivity. Preferably, said ability or failure of IL-3 to activate its receptor is determined by measuring cellular responses of IL-3 receptor positive cells.

In one embodiment, said anti-IL-3 antibody or said IL-3 binding fragment thereof is not capable of depleting IL-3 receptor expressing cells.

In one embodiment, said anti-IL-R3 antibody is obtained by a method comprising the step of immunizing a rabbit, rat, mouse, chicken, goat, guinea pig, hamster, horse, or sheep. In one embodiment, said anti-IL-3 antibody is obtained by a method comprising the step of immunizing a rabbit. In one embodiment, said anti-IL-3R antibody is obtained by a method comprising the step of immunizing a mouse.

In one embodiment, said anti-IL-3R antibody or said IL-3R binding fragment thereof binds to the human IL-3R protein. In one embodiment, said anti-IL-3R antibody or said IL-3R binding fragment thereof is specific for the human IL-3R protein. In one embodiment, said anti-IL-3R antibody or said IL-3R binding fragment thereof does not bind to IL-3R proteins of other species besides human.

In one embodiment, said anti-IL-3R antibody or said IL-3R binding fragment thereof is not capable of depleting IL-3 receptor expressing cells.

In one embodiment, said anti-IL-3 antibody or said IL-3 binding fragment thereof binds to a portion of the IL-3 amino acid sequence which portion consists of residues 22-48 of the human IL-3 amino acid sequence (SEQ ID NO: 2).

In one embodiment, said anti-IL-3 antibody or said IL-3 binding fragment thereof binds to an epitope within human IL-3 comprising or comprising a portion of the sequence defined by residues 22-48 of the human IL-3 amino acid sequence (SEQ ID NO: 2).

In one embodiment, said anti-IL-3 antibody or said IL-3 binding fragment thereof is specific for IL-3.

In one embodiment, said anti-IL-3 antibody or said IL-3 binding fragment thereof does not bind to interleukins other than IL-3.

In one embodiment, said IL-3 signaling inhibitor, preferably said anti-IL-3 antibody or said IL-3 binding fragment thereof, is administered by a route selected from the group consisting of intravenously, intramuscularly, subcutaneously, intraperitoneally, topically, orally, rectally, and inhalation administration. In one embodiment, said IL-3 signaling inhibitor, is administered intravenously, preferably by injection, or subcutaneously, preferably by injection.

In one embodiment, said IL-3 signaling inhibitor is administered daily, preferably once every day. In one embodiment, said IL-3 signaling inhibitor is administered once every week. In one embodiment, said IL-3 signaling inhibitor is administered once every two weeks. In one embodiment, said IL-3 signaling inhibitor is administered by bolus administration. In one embodiment, said IL-3 signaling inhibitor is administered for up to 5 days, preferably up to 4 days, more preferably up to 3 days, more preferably up to 2 days, more preferably 1 day. In one embodiment, said IL-3 signaling inhibitor is administered for at least one week, preferably at least two weeks, more preferably at least three weeks, more preferably at least 4 weeks, more preferably at least 8 weeks, more preferably at least 12 weeks.

In one embodiment, simultaneously with said administration of said IL-3 signaling inhibitor, preferably said anti-IL-3 antibody or IL-3 binding fragment thereof, said patient is subjected to one or more additional treatments for treating Long-COVID or Post-COVID syndrome, wherein, preferably, said treatment consists of the administration of one or more of antiinflammatory treatments.

The objects of the present invention are also solved by a pharmaceutical composition comprising at least one pharmaceutically acceptable carrier, diluent and/or excipient and an IL-3 signaling inhibitor, preferably an anti-IL-3 antibody or IL-3 binding fragment thereof, as defined above for use in the treatment of Long-COVID and/or Post-COVID syndrome.

In such pharmaceutical composition, said IL-3 signaling inhibitor and said Long-COVID and/or Post-COVID syndrome are as defined in the embodiments above.

The objects of the present invention are also solved by a method of treatment of Long-COVID and/or Post-COVID syndrome, said method comprising administration of an effective amount of an IL-3 signaling inhibitor, preferably an anti-IL-3 antibody or an IL-3 binding fragment thereof to a patient in need thereof.

In such method, said IL-3 signaling inhibitor, preferably said anti-IL-3 antibody or said IL-3 binding fragment thereof, said Long-COVID or Post-COVID syndrome and said administration are as defined in the embodiments herein.

The objects of the present invention are also solved by the use of an IL-3 signaling inhibitor, preferably an anti-IL-3 antibody or an IL-3 binding fragment thereof, in the manufacture of a medicament for the treatment of Long-COVID or Post-COVID syndrome.

In one embodiment, said treatment occurs by administration of said IL-3 signaling inhibitor to a patient in need thereof.

In such use and the embodiment referring to it, said IL-3 signaling inhibitor, said Long-COVID and/or Post-Covid syndrome, and said administration are as defined in the embodiments herein.

The amino acid sequence of the human IL-3 amino acid sequence without the signal peptide is provided by SEQ ID NO: 1.

The amino acid sequence of residues 22-48 of human IL-3 amino acid sequence (without the signal peptide) is provided by SEQ ID NO: 2.

The amino acid sequence of the V_{H} region of the monoclonal antibody P8C11 is provided by SEQ ID NO: 3.

The amino acid sequence of the HCDR1 region of the monoclonal antibodies P8C11 and GRT002-H16L2 is provided with SEQ ID NO: 4, as defined by Kabat numbering.

The amino acid sequence of the HCDR2 region of the monoclonal antibodies P8C11 and GRT002-H16L2 is provided with SEQ ID NO: 5, as defined by Kabat numbering.

The amino acid sequence of the HCDR3 region of the monoclonal antibodies P8C11 and GRT002-H16L2 is provided with SEQ ID NO: 6, as defined by Kabat numbering.

The amino acid sequence of the HCDR1 region of the monoclonal antibodies P8C11 and GRT002-H16L2 is provided with SEQ ID NO: 7, as defined by IMGT numbering.

The amino acid sequence of the HCDR2 region of the monoclonal antibodies P8C11 and GRT002-H16L2 is provided with SEQ ID NO: 8, as defined by IMGT numbering.

The amino acid sequence of the HCDR3 region of the monoclonal antibodies P8C11 and GRT002-H16L2 is provided with SEQ ID NO: 9, as defined by IMGT numbering.

The amino acid sequence of the V_{L} region of the monoclonal antibody P8C11 is provided by SEQ ID NO: 10.

The amino acid sequence of the LCDR1 region of the monoclonal antibodies P8C11 and GRT002-H16L2 is provided with SEQ ID NO: 11, as defined by Kabat numbering.

The amino acid sequence of the LCDR2 region of the monoclonal antibodies P8C11 and GRT002-H16L2 is provided with SEQ ID NO: 12, as defined by Kabat numbering.

The amino acid sequence of the LCDR3 region of the monoclonal antibodies P8C11 and GRT002-H16L2 is provided with SEQ ID NO: 13, as defined by Kabat numbering.

The amino acid sequence of the LCDR1 region of the monoclonal antibodies P8C11 and GRT002-H16L2 is provided with SEQ ID NO: 14, as defined by IMGT numbering.

The amino acid sequence of the LCDR2 region of the monoclonal antibodies P8C11 and GRT002-H16L2 is provided with SEQ ID NO: 15, as defined by IMGT numbering. The LCDR2 region comprises the three amino acid "KAS".

The amino acid sequence of the LCDR3 region of the monoclonal antibodies P8C11 and GRT002-H16L2 is provided with SEQ ID NO: 16, as defined by IMGT numbering.

The amino acid sequence of the V_{H} region of the monoclonal antibody GRT002-H16L2 is provided by SEQ ID NO: 17.

The amino acid sequence of the V_{L} region of the monoclonal antibody GRT002-H16L2 is provided by SEQ ID NO: 18.

The amino acid sequence of the entire V_{H} region of the monoclonal antibody GRT002-H16L2 is provided by SEQ ID NO: 19.

The amino acid sequence of the entire V_{L} region of the monoclonal antibody GRT002-H16L2 is provided by SEQ ID NO: 20.

### BRIEF DESCRIPTION OF THE FIGURES

The present invention is now further described by reference to the following figures.

All methods mentioned in the figure descriptions below were carried out as described in detail in the examples.
**Figure 1****: Gating strategy of single cells and T cells.**
**Figure 2****: Absolute T cell counts and CD4⁺ T cell phenotype of patients with Long-**COVID compared to controls and healthy controls.
   A) Absolute CD4⁺ and CD8⁺ T cells counts in fresh whole blood from 69 healthy controls (Healthy), 23 controls and 68 patients with Long-COVID (L-COV) were analyzed. Each sample is represented by one dot, and the mean is shown as bar. One-way ANOVA with Bonferroni's multiple comparison test was used. B) Percentage of naive (Naive), effector-memory (EM) and terminal-effector-memory (TEMRA) CD4⁺ T cells in fresh blood of 54 Long-COVID patients. Long-COVID patients were stratified according the severity of symptoms into Long-COVID-Score 1 (n=8), Score 2 (n=30) and Score 3 (n=16).

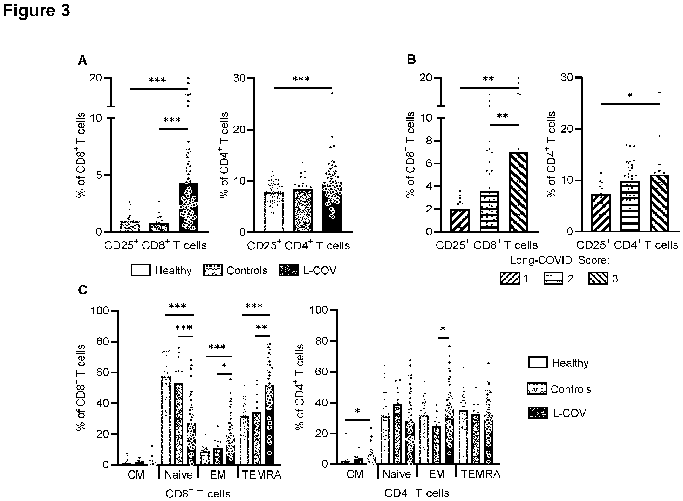

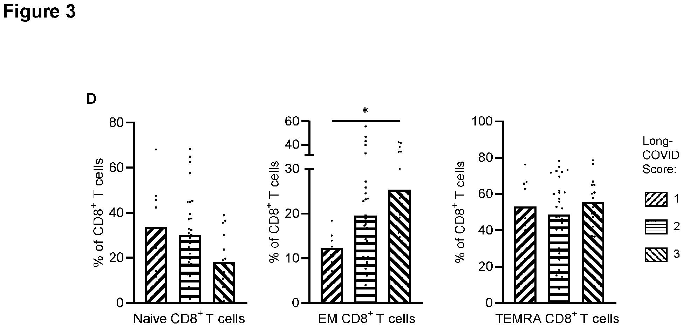

**Figure 3****: T cell phenotype of patients with Long-COVID compared to controls and healthy controls.**
   **A), B)** Percentage of CD25 expressing CD8⁺ or CD4⁺ T cells in fresh blood of 69 healthy controls (Healthy), 23 controls and 68 Long-COVID patients (L-COV). Long-COVID patients were stratified according the severity of symptoms into Long-COVID-Score 1 (n=11), Score 2 (n=38) and Score 3 (n=19). Each sample is represented by one dot and the mean is shown as bar. **C, D)** Percentage of central-memory (CM), naive (Naive), effector-memory (EM) and terminal-effector-memory (TEMRA) CD8⁺ or CD4⁺ T cells in fresh blood of 30 healthy controls, 11 controls and 54 Long-COVID patients. Long-COVID patients were stratified according the severity of symptoms into Long-COVID-Score 1 (n=8), Score 2 (n=30) and Score 3 (n=16). Each sample is represented by one dot and the mean is shown as bar. One-way ANOVA with Bonferroni's multiple comparison test was used.

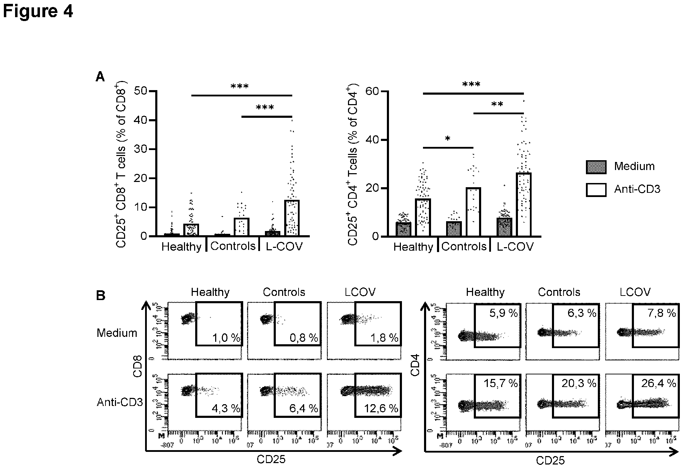

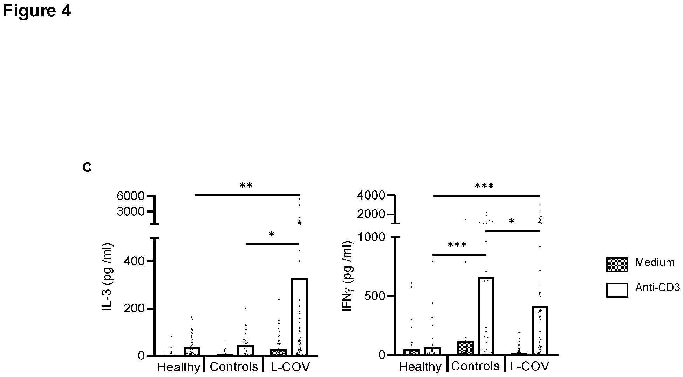

**Figure 4****: T cell reactivity in patients with Long-COVID compared to controls and healthy controls.**
   **A-C)** Whole-blood samples from 69 healthy controls (Healthy), 23 controls and 68 patients with Long-COVID (L-COV) were cultured with or without immobilized anti-CD3 antibodies for 24h. **A, B)** Expression of CD25 was quantified by flow cytometry on CD8⁺ T cells and CD4⁺ T cells. Each sample is represented by one dot, and the mean is shown as bar. Representative dot plots are shown. **C)** Concentrations of IL-3 and IFN-γ were measured in the culture supernatant by ELISA from 48 healthy controls (Healthy), 23 controls and 68 patients with Long-COVID (L-COV). Each sample is represented by one dot, and the mean is shown as bar. One-way ANOVA with Bonferroni's multiple comparison test was used.

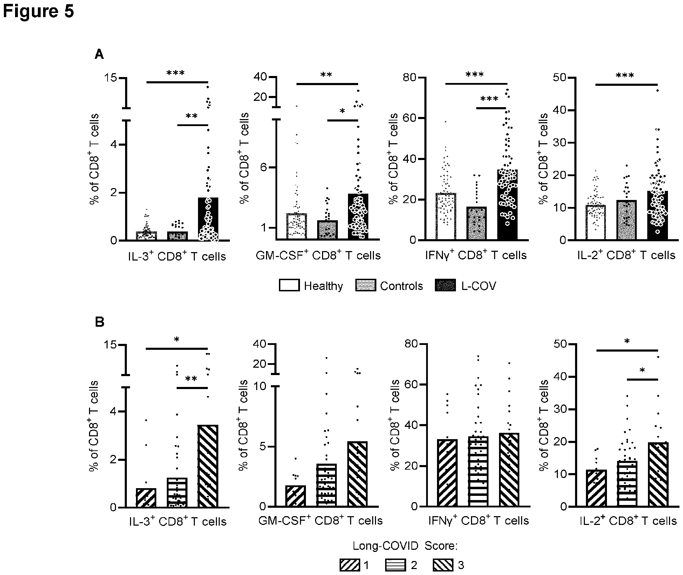

**Figure 5****: Intracellular cytokine expression of CD8⁺ T cells in patients with Long-COVID compared to controls and healthy controls.**
   **A-B)** PBMCs from 69 healthy controls (Healthy), 23 controls (Controls) and 68 patients with Long-COVID (L-COV) were activated with PMA/Ionomycin in the presence of brefeldin A for 3 hours. Long-COVID patients were stratified according the severity of symptoms into Long-COVID-Score 1 (n=11), Score 2 (n=38) and Score 3 (n=19). Intracellular cytokine expression by CD8⁺ T cells was analyzed by flow cytometry. Each sample is represented by one dot, and the mean is shown as bar. One-way ANOVA with Bonferroni's multiple comparison test was used.

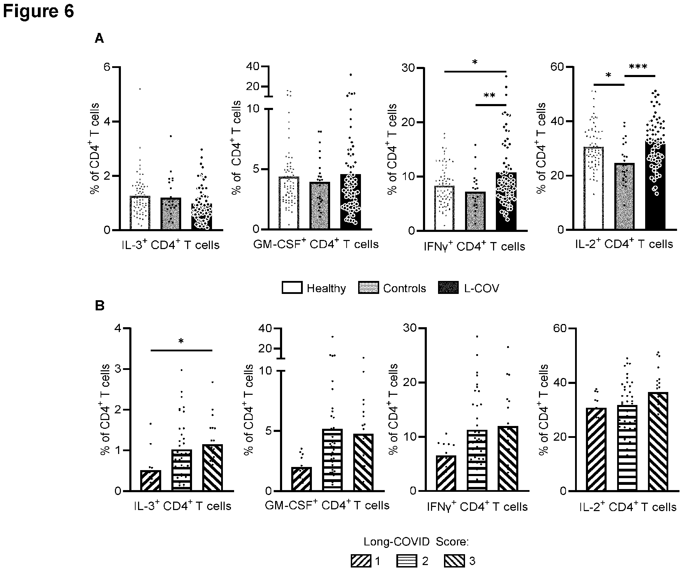

**Figure 6****: Intracellular cytokine expression of CD4⁺ T cells in patients with Long-COVID compared to controls and healthy controls.**
   **A-B)** PBMCs from 69 healthy controls (Healthy), 23 controls and 68 patients with Long-COVID (L-COV) were activated with PMA/Ionomycin in the presence of brefeldin A for 3 hours. Long-COVID patients were stratified according the severity of symptoms into Long-COVID-Score 1 (n=11), Score 2 (n=38) and Score 3 (n=19). Intracellular cytokine expression by CD4⁺ T cells was analyzed by flow cytometry. Each sample is represented by one dot, and the mean is shown as bar. One-way ANOVA with Bonferroni's multiple comparison test was used.

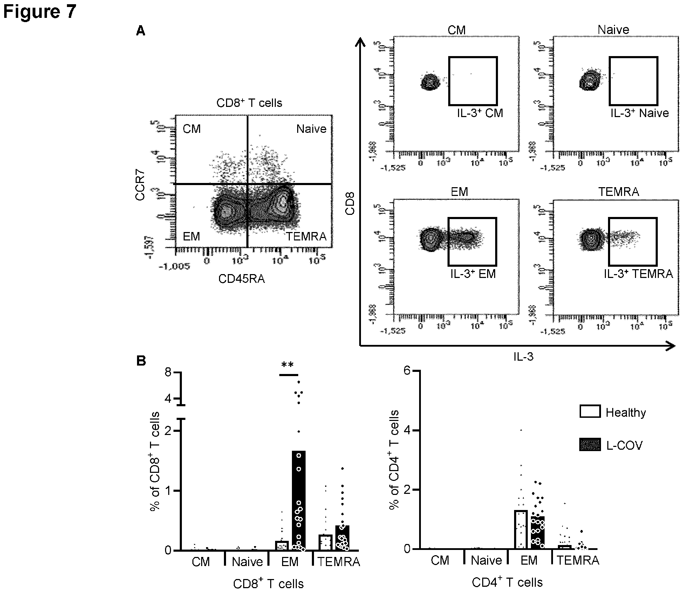

**Figure 7****: Long-COVID patients show elevated IL-3 expression by** CD8⁺ **memory T cells compared to healthy controls.**
   **A-B)** PBMCs from 19 healthy controls (Healthy) and 20 patients with Long-COVID (L-COV) were activated with PMA/Ionomycin in the presence of brefeldin A for 3 hours. Intracellular IL-3 expression by central-memory (CM), naive (Naive), effector-memory (EM) and terminal-effector-memory (TEMRA) CD8⁺ and CD4⁺ T cells was analyzed by flow cytometry. **A)** Representative dot plots for CD8⁺ T cells. **B)** Each sample is represented by one dot, and the mean is shown as bar. Two-tailed student's t-test was used to calculate the significance between patients with Long-COVID and healthy controls.

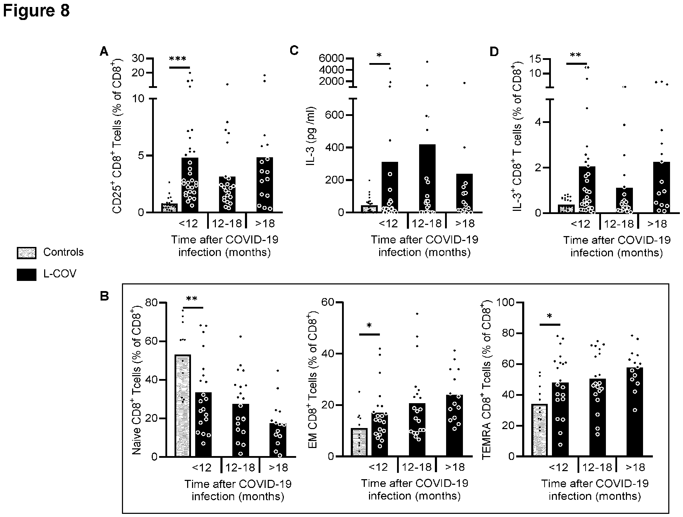

**Figure 8****: Persisting T cell hyper-activation of Long-COVID patients for up to 18 months after initial SARS-CoV-2 infection.**
   **A-D)** Peripheral blood from 23 controls and 68 patients with Long-COVID (L-COV) was analyzed. Controls and patients with Long-COVID were stratified according to the time after the initial SARS-CoV-2 infection in < 12 months (Controls n=23, L-COV n=30), 12-18 months (L-COV n=22), >18 months (L-COV n=16). **A)** Percentage of CD25 expressing CD8⁺ T cells. **B)** Percentage of central memory (CM), naive (Naive), effector-memory (EM) and terminal-effector-memory (TEMRA) CD8⁺ T cells. **C)** Concentration of IL-3 in the supernatant of whole-blood samples cultured with immobilized anti-CD3-antibodies for 24h. **D)** Intracellular expression of IL-3 by CD8⁺ T cells. Each sample is represented by one dot, and the mean is shown as bar. One-tailed Welch's t-test was used to calculate the significance between patients with Long-COVID patients and controls.

In the following, reference is made to the examples, which are given to illustrate, not to limit the present invention.

### EXAMPLES

### Example 1

### Study subjects and sampling

A total of 68 adult patients diagnosed with Long-COVID syndrome, 23 controls and 69 adult healthy controls without any history of SARS-CoV-2 infection were enrolled at a unit specialized for Long-COVID at the Klinik Donaustauf, Germany, from Nov 2021 to July 2022. This study was approved by the Research Ethics Committee from the University Hospital Regensburg (Study and Approval Number: 20-1785-101). Informed consent was obtained from all participants.

Post-COVID syndrome was defined as a condition that occurs in individuals with a history of confirmed SARS-CoV-2 infection, ≥3 months from the onset of infection with symptoms that last for at least 2 months and cannot be explained by an alternative diagnosis. Symptoms could either be new onset, following initial recovery from an acute SARS-CoV-2 infection, or persist from the initial illness. In the present study, all patients suffering from Long-COVID also fell under the definition of patients suffering from Post-COVID syndrome. Thus, for the Examples of the present invention, expressions such as "Long-COVID cohort", "Long-COVID patients", or "patients with Long-COVID" are to be understood to be interchangeable with "Post-COVID syndrome cohort", "Post-COVID syndrome patients", or "patients with Post-COVID syndrome", respectively.

Patients in the matched control group had a history of confirmed SARS-CoV-2 infection but no clinical symptoms. The Long-COVID cohort was classified based on self-assessment of the severity of their illness using the Long-COVID severity score as described by Kick *et al.,* 2020¹ using the following scores: 0 = no limitations in everyday life and no symptoms, pain, depression or anxiety related to the infection, 1 = negligible limitations in everyday life, performance of all usual duties/activities, however with persistent symptoms, pain, depression or anxiety related to the infection, 2 = limitations in everyday life with occasionally need to avoid or reduce usual duties/activities or to spread these over time due to symptoms, pain, depression or anxiety related to the infection. Patients are able to perform all activities without any assistance. 3 = limitations in everyday life with inability to perform all usual duties/activities due to symptoms, pain, depression or anxiety related to the infection. Patients are able to take care of themselves without any assistance. 4 = severe limitations in everyday life with inability to take care of themselves and dependence on nursing care and/or assistance from other persons due to symptoms, pain, depression or anxiety related to the infection.

Fresh whole blood was available from 68 healthy controls without a previous history of SARS-CoV-2 infection, 23 controls without persistent symptoms following SARS-CoV-2 infection and 68 Long-COVID patients for immediate immunophenotyping by flow cytometry with at least one antibody panel and for the whole blood stimulation assay. Peripheral blood mononuclear cells (PBMCs) were prepared from 69 healthy controls, 23 controls and 68 Long-COVID patients. For subgroup analysis the Long-COVID patients were stratified according the Long-COVID-Score into the groups "1" (11 patients), "2" (38 patients) and "3" (19 patients). Further antibody panels were used in subsets of patients with sample numbers for each readout provided in the figure legends, as for some read-outs not enough material was available.

### Whole blood stimulation assay

FACS-tubes (BD Bioscience) were pre-coated with 300 µl RPMI-1640 (Gibco, Karlsruhe, Germany) containing 5 µg/ml anti-CD3 antibodies (OKT-3, eBioscience, San Diego, USA) or just with 300 µl RPMI-1640 medium at 37° C for 4 hours and washed twice with 4 ml 0,9% NaCl. After the last washing step 300 µl RPMI 1640 containing 1% penicillin/streptomycin and 10% heat-inactivated FCS was added, tubes were sealed and stored frozen by -20° C up to 4 weeks until use. For T cell activation assays, pre-coated FACS-tubes were thawed by room temperature and 100 µl medium were removed. 100 µl of heparinized blood were added to pre-coated tubes and incubated at 37° C in 5% CO₂ for 24 hours. The supernatants were recovered and released cytokines analyzed by ELISA. Cells were analyzed by multi-parametric flow cytometry as described below.

### Flow cytometry

Heparin-anticoagulated fresh blood samples (100 µl) or cells after whole blood stimulation were incubated with various panels of the following directly labeled monoclonal antibodies for 20 minutes by 4° C: anti-CD3 FITC (clone: OKT3, BioLegend, San Diego, CA), anti-CD3 (clone: SK7, BioLegend), anti-CD4 PerCP-Cyanine5.5 and APC (clone: RPA-T4, BioLegend), anti-CD4 BD Horizon V500 (clone: RPA-T4, BD Bioscience), anti-CD8a APC-Cyanine7 and PerCP-Cyanine5.5 (clone: RPA-T8, BioLegend), anti-CD16 Pacific Blue (clone: 3G8, BioLegend), anti-CD25 APC (clone: BC96, BioLegend), anti-CD45RA Brilliant Violett 510 (clone: HI100, BioLegend), anti-CD197/CCR7 APC (clone: 7-239, BioLegend).

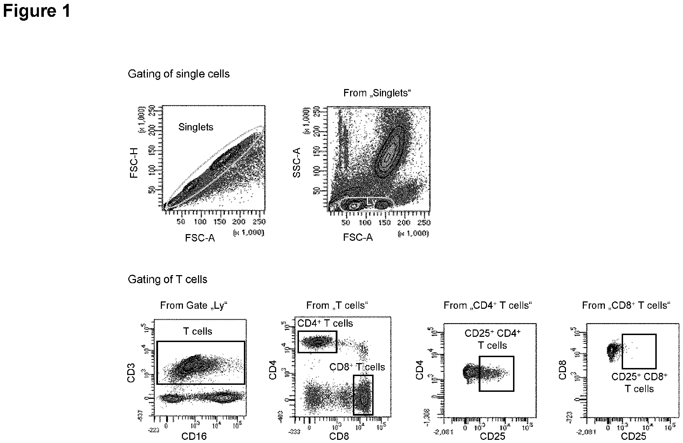

Subsequently, samples were treated with FACS Lysing Solution (BD Bioscience) for 10 min, washed with 0,9% NaCl, centrifuged, resuspended in 0,9% NaCl together with FACS counting beads (Invitrogen, Darmstadt, Germany) and acquired with a BD FACSCanto II flow cytometer and analyzed with FACSDIVA 8 software (BD Biosciences). The gating strategy for key cell populations is shown in **Figure 1****.**

### Preparation of PBMCs

Peripheral blood mononuclear cells (PBMCs) were prepared by Ficoll-Paque density gradient centrifugation from heparin-anticoagulated fresh blood samples. For cryopreservation, PBMCs were resuspended in fetal calf serum (FCS) with 10% dimethylsulfoxide (DMSO) at a concentration of 1-2 × 10⁶ cells / ml, frozen at -80° C for 2-3 days and then transferred into liquid nitrogen. The cells were thawed in a 37° C water bath and washed 3 times with medium. The viability was controlled by Trypan-blue staining and was 90-95%.

### Intracellular cytokine staining

For intracellular staining of cytokines 500.000 PBMC/well were cultured in 200 µl RPMI 1640 medium (Gibco, Karlsruhe, Germany) containing 1% penicillin/streptomycin (Gibco, Karlsruhe, Germany), 10% fetal calf serum, 1% L-glutamine (Gibco, Karlsruhe, Germany) and Phorbol-12-myristat-13-acetat (PMA, 10 ng/ml), ionomycin (1 µg/ml) and brefeldin A (5 µg/ml) (eBioscience) for 3 hours by 37° C. After extracellular staining anti-CD3-APC-Cy7 (clone: SK7, BioLegend), anti-CD4-APC (clone: RPA-T4, BioLegend), anti-CD8a-PerCP-Cy5.5 (clone: RPA-T8, BioLegend) cells were treated with Fix-Perm and Perm-Wash solutions (BD Biosciences) incubated and stained intracellularly with the following antibodies: anti-IL-2 PECy7 (clone: MQ1-17H12, BioLegend), anti-IL-3 PE (clone: clone-13, AG Mack) or mouse IgG1, x Isotype Ctrl PE (clone: MOPC-21, BioLegend), anti-GM-CSF Pacific Blue (clone: BVD2-21C11, BioLegend), anti-IFN-γ FITC (clone: 4S.B3, BioLegend).

### Enzyme Linked Immuno Sorbent Assay (ELISA)

Concentration of IFN-γ in whole blood assay supernatants was measured by ELISA, according to manufacturer's protocol (DuoSet Elisa, R&D Systems, Abington, UK). For measurement of IL-3, ELISA plates (NUNC Maxisorb, Thermofisher Scientific, Waltham, USA) were coated with a capture anti-IL-3 antibody (Clone P8C11, AG Mack; 5 µg/ml in PBS) in 100 µl / well at room temperature (RT) overnight. Plates were washed 3 times with PBS / 0.05% Tween-20, blocked with PBS containing 1% bovine serum albumin (BSA) at RT for 1 hour and washed again with PBS. Samples where preincubated with 100 µg/ml mouse IgG1, kappa isotype control antibody (MOPC21, BioXCell, Lebanon, USA) for 1 hour by RT and added to the plates for 2 hours by RT (100 µl / well, diluted in PBS / 1% BSA). Recombinant human IL-3 (BioLegend) was diluted from 7.8 - 500 pg/ml in PBS / 1% BSA and served as standard. After washing plates were incubated with 400 ng/ml HRP-labelled detection anti-IL-3 antibody (Clone 13, AG Mack) (100 µl/well) for 1.5 hours at RT and color reaction was performed with TMB Substrate Solution (BioLegend) according to manufacturer's protocol.

### Statistical analysis

Statistical analysis was performed using the GraphPad Prism 8 Software. Statistical differences between more than two different cell stimulations or patient cohorts were calculated using one-way ANOVA with Bonferroni's multiple comparison as indicated in the figures. For two group comparisons, 2-tailed unpaired t-test was used as indicated in the figures. P values less than 0.05 were considered significant and marked with one asterisk or with two asterisks if less than 0.01. P values less than 0.001 were marked with three asterisks.

### Example 2

All methods mentioned in this example were carried out as described in Example 1.

Immunophenotyping was performed of fresh whole blood from patients with Long-COVID, controls without Long-COVID, and healthy donors. Long-COVID patients were diagnosed according to established criteria for Long-COVID as defined in Example 1. Controls had a previous SARS-CoV-2 infection without any persistent symptoms, while healthy controls had no history of SARS-CoV-2 infection.

Characteristics of Long-COVID patients are shown in Table 1 and reveal typical clinical symptoms like fatigue, stress dyspnea, chest pain, poor concentration, impaired sleep and depression. Patients were stratified according to the severity of their symptoms using a Long-COVID-Score ranging from 1 to 3. A score of 3 was associated with more severe initial SARS-CoV-2 infection as seen by a higher frequency of hospitalization and ventilation and with more comorbidities (**Table 1**).

**Table 1:**

| | **Healthy** | **Controls** | **Long-COVID all** | **Long-COVID Score 1** | **Long-COVID Score 2** | **Long-COVID Score 3** |
|---|---|---|---|---|---|---|
| Number of patients (n) | 69 | 23 | 68 | 11 | 38 | 19 |

| **Demographics:** | | | | | | |
|---|---|---|---|---|---|---|
| Mean age (Years, min-max) | 26.9 (21-82) | 27.5 (23-45) | 50.6 (24-72) | 51.2 (31-61) | 48.6 (24-64) | 54.5 (37-72) |
| Sex (% female) | 47 (68%) | 10 (43.5%) | 45 (66 %) | 8 (72.7%) | 28 (73.7%) | 9 (47.4%) |

| **Initial COVID-19 infection:** | | | | | | |
|---|---|---|---|---|---|---|
| Time after infection (months) | | 4.7 | 13.1 | 14.8 | 12.0 | 14.8 |
| Hospitalization n (%) | | 0 | 11 (16.2%) | 0 (0%) | 6 (15.8%) | 5 (26.3%) |
| ICU n (%) | | 0 | 5 (7.4%) | 0(0%) | 2 (5.3%) | 3 (15.8%) |
| Ventilation n (%) | | 0 | 4 (5.9%) | 0 (0%) | 1 (2.6%) | 3 (15.8%) |

| **Long-COVID symptoms n (%):** | | | | | | |
|---|---|---|---|---|---|---|
| Fatigue | | | 56 (82.4%) | 5 (45.5%) | 36 (94.7%) | 15 (78.9%) |
| Stress dyspnea | | | 55 (80.9%) | 8 (72.7%) | 32 (84.2%) | 15 (78.9%) |
| Cough | | | 6 (8.8%) | 0 (0%) | 4 (10.5%) | 2 (10.5%) |
| Chest Pain / Tightness | | | 18 (26.5%) | 2 (18.2%) | 10 (26.3%) | 6 (31.6%) |
| Taste impairment | | | 16 (23.5%) | 1 (9.1%) | 10 (26.3%) | 5 (26.3%) |
| Smelling impairment | | | 16 (23.5%) | 0 (0%) | 11 (28.9%) | 5 (26.3%) |
| Poor concentration | | | 56 (82.4%) | 8 (72.7%) | 32 (84.2%) | 16 (84.2%) |
| Affected Sleep | | | 37 (54.4%) | 3 (27.3%) | 22 (57.9%) | 12 (63%) |
| Joint / Muscles pain | | | 32 (47.1%) | 4 (36.4%) | 19 (50%) | 9 (47.4%) |
| Palpitations | | | 17 (25%) | 1 (9.1%) | 12 (31.6%) | 4 (21.1%) |
| Dizziness | | | 13 (19.1%) | 2 (18.2%) | 7 (18.4%) | 4 (21.1%) |
| Headache / Migraine | | | 15 (22.1%) | 1 (9.1%) | 8 (21.1%) | 6 (31.6%) |
| Depression | | | 28 (41.2%) | 0 (0%) | 18 (47.4%) | 10 (52.6%) |

| **Comorbidities n (%):** | | | | | | |
|---|---|---|---|---|---|---|
| Smoking | | | 8 (11.8%) | 1 (9.1%) | 2 (5.3%) | 5 (26.3%) |
| Cardiovascular disease | | | 2 (2.9%) | 0(0%) | 0(0%) | 2 (10.5%) |
| Lung disease | | | 13 (19.1%) | 3 (27.3%) | 5 (13.2%) | 5 (26.3%) |
| Immunosuppression | | | | 0 (0%) | 1 (2.6%) | 2 (10.5%) |

| **Lung function n (%):** | | | | | | |
|---|---|---|---|---|---|---|
| Restriction | | | 3 (4.4%) | 0 (0%) | 0 (0%) | 3 (15.8%) |
| Impaired diffusion | | | 7 (10.3%) | 0 (0%) | 3 (7.9%) | 4 (21.1%) |
| FEV1 (%) | | | 92.3 | 92.9 | 95.6 | 85.6 |
| TLCO (%) | | | 89.2 | 90.2 | 91.5 | 84.3 |

| **Echocardiography n (%):** | | | | | | |
|---|---|---|---|---|---|---|
| Impaired LVEF | | | 3 (4.4%) | 0 (0%) | 1 (2.6%) | 2 (10.5%) |

| **6-min walking test n (%):** | | | | | | |
|---|---|---|---|---|---|---|
| Distance (% debit) | | | 87.6% | 90.7% | 91.9% | 77.5% |
| Borg-CR10-Scale | | | 4.5 | 4.4 | 4.2 | 5.3 |

Only in a small fraction of the patients, objective investigational findings became evident with impaired lung function consisting of restriction and impaired diffusing capacity as well as reduced left-ventricular ejection fraction and impaired performance in the 6-min walk. These changes were more prominent in patients with a Long-COVID-Score of 3.

### Example 3

All methods mentioned in this example were carried out as described in Example 1.

CD8⁺ T cells are more activated and show a memory penotype in patients with Long-COVID.

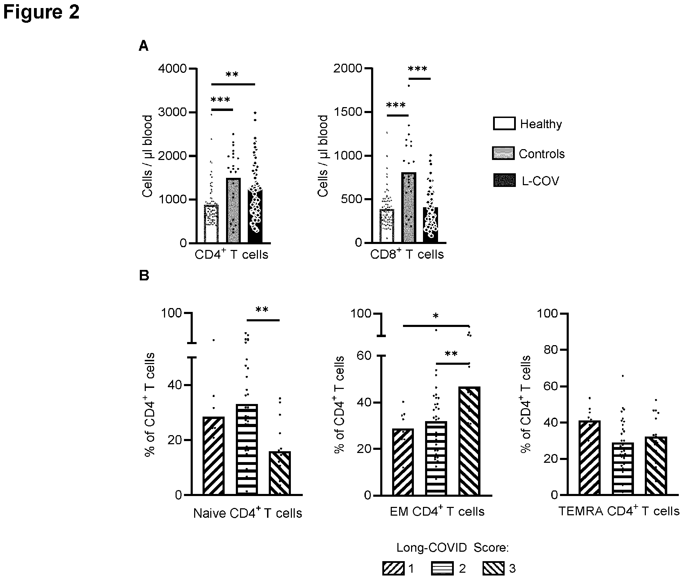

Total numbers of CD4⁺ and CD8⁺ T cells were almost identical in healthy controls and Long-COVID patients (**Figure 2A**). Controls showed somewhat higher numbers of T cells most likely due to the shorter interval between SARS-CoV-2 infection and blood analysis. Immunophenotyping revealed a highly significant and 7-fold higher frequency of activated CD8⁺ T cells in patients with Long-COVID as shown by surface expression of CD25 (**Figure 3A****)**. The percentage of activated CD8⁺ T cells also strongly correlated with the Long-COVID-Score (**Figure 3B****)**. Among CD4⁺ T cells, only a minor expansion of CD25⁺ cells and no clear correlation with the Long-COVID-Score was found (**Figure 3A**, B). However, one has to consider that in CD4⁺ T cells expression of CD25 is not specific for activated cells, but also present on regulatory cells.

Subgrouping of T cells into naive, central-memory, effector-memory, and terminal-effector cells according to their expression of CCR7 and CD45RA revealed an about 2-fold increased fraction of effector-memory and terminal-effector CD8⁺ T cells and a decreased fraction of naive CD8⁺ T cells in Long-COVID patients (**Figure 3C**). The expansion of effector-memory CD8⁺ T cells was most pronounced in patients with a Long-COVID-Score of 3 (**Figure 3D**). For CD4⁺ T cells these changes were absent or less clear (**Figure 3C** and **Figure 2B**).

### Example 4

All methods mentioned in this example were carried out as described in Example 1.

T cells from patients with Long-COVID are more susceptible to activation and release larger amounts of cytokines.

Fresh whole blood was incubated with immobilized anti-CD3 antibodies for 24 hours to induce polyclonal activation of T cells. Upregulation of CD25 was detected by flow cytometry and the percentage of CD25⁺ T cells was analyzed. CD8⁺ T cells from patients with Long-COVID showed a more pronounced upregulation of CD25 after polyclonal stimulation. CD25 expression was induced on 12.6% of the CD8⁺ T cells from Long-COVID while only 4.0% and 6.4% of the CD8⁺ T cells from healthy controls and controls upregulated CD25 (**Figure 4A**, **B**). Similar but less pronounced changes were seen for CD4⁺ T cells (**Figure 4A**, **B**).

The release of T cell derived cytokines in the supernatant of whole blood stimulated with immobilized anti-CD3 antibodies was also quantified, choosing cytokines that were previously found to be altered in patients with acute COVID-19 infection. In contrast to healthy controls and controls, high amounts of IL-3 in the supernatant of whole blood from Long-COVID patients were detected (**Figure 4C**). The release of IFN-γ was increased in both, controls and Long-COVID patients, indicating that an increased release of IFN-γ is more a consequence of a recent SARS-CoV-2 infection and not necessarily associated with development of Long-COVID (**Figure 4C**).

### Example 5

All methods mentioned in this example were carried out as described in Example 1.

Intracellular staining shows increased cytokine expression in CD8+ effector-memory T cells.

To find out which subsets of T cells are responsible for the increased release of cytokines in Long-COVID, intracellular cytokine staining after stimulation with PMA/Ionomycin in the presence of brefeldin-A for 3h was performed.

An about 5-fold higher percentage of CD8⁺ T cells expressing IL-3 in Long-COVID patients compared to healthy controls and controls was found (**Figure 5A**), while expression of GM-CSF, IFN-γ and IL-2 was much less upregulated in Long-COVID patients. Intracellular staining of IL-3 and IL-2 strongly correlated with the Long-COVID-Score (**Figure 5B**). The majority of patients with a Long-COVID-Score of 3 had clearly elevated counts of IL-3⁺ CD8⁺ T cells. CD4⁺ T cells did not show a convincing overexpression of cytokines in Long-COVID (**Figure 6**), indicating that hyper-activity and hyper-reactivity in patients with Long-COVID is largely restricted to CD8⁺ T cells.

To more specifically identify the subset of IL-3 producing CD8⁺ T cells in patients with Long-COVID these T cells were further characterized according to their memory status. Expression of IL-3 was predominately found in effector-memory (EM) and to lower degree in terminal-effector-memory (TEMRA) cells of patients with Long-COVID as shown in the dot plots and the statistical analysis (**Figure 7A**, **B**). In contrast, almost no IL-3 expression was seen in central-memory and naive CD8⁺ T cells. Healthy controls showed a much lower IL-3 expression in effector-memory CD8⁺ T cells. CD4⁺ T cell memory subsets showed no differences in IL-3 expression between Long-COVID and healthy controls (**Figure 7B**).

Thus, Long-COVID patients did not only show an increased frequency of EM and TEMRA CD8⁺ T cells (**Figure 3C**) but also an increased percentage of EM and TEMRA CD8⁺ T cells expressing IL-3.

### Example 6

All methods mentioned in this example were carried out as described in Example 1.

Phenotypic changes of CD8⁺ T cells persist for up to 18 months after SARS-Cov-2 infection in patients with Long-COVID.

To find out whether the above described phenotypic changes gradually disappear with a longer duration of Long-COVID, the cohort of the previous Examples was stratified into patients with symptoms <12 months after infection, 12 to 18 months after infection and more than 18 months after infection. All controls were analyzed <12 months after infection and are also included in the analysis. As shown in **Figure 8A****,** the high fraction of CD25⁺ CD8⁺ T cells did not disappear in patients with a longer duration of Long-COVID. Regarding the memory status of CD8⁺ T cells, there was even some increase of effector-memory and TEMRA cells over time and a concomitant decrease of naive CD8⁺ T cells (**Figure 8B**). IL-3 expression and the percentage of IL-3⁺ CD8+T cells remained high more than 18 months after infection in patients with Long-COVID (**Figure 8C****, D**). Overall, these data suggest that CD8⁺ T cell hyper-activation and hyper-reactivity is persistent in Long-COVID patients.

### Example 7

The previous Examples demonstrate the importance of IL-3 signaling in the development and persistence of Long-COVID or Post-COVID syndrome. This was not to be expected by the prior art. Previous studies demonstrated a pronounced T cell hypo-reactivity in patients with severe acute SARS-CoV-2 infection ². To examine whether these changes could persist in Long-COVID, in the previous Examples, T cell numbers and functions in patients with Long-COVID, controls without Long-COVID and healthy volunteers were analyzed.

The inventors of the present invention surprisingly show that the opposite is the case as a marked hyper-reactivity of T cells in Long-COVID is detected. Hyper-activation is evident by a higher percentage of CD8⁺ T cells expressing the activation marker CD25, a stronger upregulation of CD25 after polyclonal stimulation, a stronger release of cytokines especially IL-3 and a higher fraction of memory T cells. These changes are more pronounced in patients with a higher Long-COVID severity score and persist for up to 18 months. Moreover, the hyper-reactivity is much more pronounced in CD8⁺ T cells compared to CD4⁺ T cells. More specifically, effector-memory and terminal-effector-memory CD8⁺ T cells are expanded in Long-COVID and express more IL-3. Most of the IL-3 is expressed by effector-memory T cells in patients with Long-COVID.

A pronounced T cell hyper-reactivity is seen even 18 months after initial SARS-CoV-2 infection with little decrease over time suggesting that counter regulatory mechanisms leading to contraction of immune responses are insufficient in Long-COVID.

Some T cell alterations have already been described in patients with Long-COVID including more IL-2 and IL-6 producing T cells, reduced naive T cell subsets, T cell senescence, a more rapid decline of SARS-CoV-2 specific memory CD8⁺ T cells and increased serum levels of interferons and pro-inflammatory cytokines, IL-1β, TNF-alpha and IL-6. However, in most studies, patients were only stratified by the severity of the initial SARS-CoV-2 infection and not by the severity or duration of their Long-COVID symptoms. None of the study analyzed expression of IL-3, which best distinguishes Long-COVID from controls in the patient cohort of the present Examples.

Overall, the above results demonstrate an involvement of CD8⁺ T cells in the development of Long-COVID. The increased release of IL-3 by hyper-activated and hyper-reactive T cells correlates surprisingly well with the clinical symptoms of Long-COVID similar to the chronic fatigue syndrome in patients with autoimmunity. The data clearly indicate that T cell-directed immunosuppressive therapies or interference with T cell-derived effector molecules, especially IL-3, will be of benefit for patients with Long-COVID.

As demonstrated by the previous Examples, the differences between controls and Long-COVID patients are most pronounced for IL-3 as seen by the release of IL-3 in the supernatant and by intracellular cytokine staining of CD8⁺ T cells.

Thus, inhibiting the IL-3 signaling pathway with an IL-3 signaling inhibitor according to the present invention will help to treat Long-COVID and/or Post-COVID syndrome in patients.

The features of the present invention disclosed in the specification, the claims, and/or in the accompanying figures may, both separately and in any combination thereof, be material for realizing the invention in various forms thereof.

### REFERENCES

1. Klok, F.A. et al. The Post-COVID-19 Functional Status scale: a tool to measure functional status over time after COVID-19. Eur Respir J 56 (2020).
2. Renner, K. et al. Severe T cell hyporeactivity in ventilated COVID-19 patients correlates with prolonged virus persistence and poor outcomes. Nat Commun 12, 3006 (2021).

## Claims

1. An IL-3 signaling inhibitor for use in a method of treatment of Long-COVID and/or Post-COVID syndrome after infection with a coronavirus in a patient in need thereof, wherein, preferably, the coronavirus is SARS-CoV-2 or a variant thereof.

2. The IL-3 signaling inhibitor for use according to claim 1, wherein the patient is a human or an animal, preferably a human.

3. The IL-3 signaling inhibitor for use according to claim 1 or 2, wherein said Long-COVID and/or Post-COVID syndrome is accompanied by an increased IL-3 expression compared to a healthy state.

4. The IL-3 signaling inhibitor for use according to any of the foregoing claims, wherein the IL-3 signaling inhibitor is selected from a group comprising antibodies including bispecific antibodies and nanobodies, fragments of said antibodies capable of binding an antigen, fusion proteins, antibody prodrugs, oligopeptides, small molecule inhibitors, oligonucleotides, such as aptamers, siRNAs, and shRNAs, and nucleic acids such as DNA and RNA, encoding said antibodies including bispecific antibodies and nanobodies, fragments of said antibodies capable of binding an antigen, fusion proteins, antibody prodrugs, oligopeptides, and oligonucleotides, such as aptamers, siRNAs, and shRNAs;
wherein, preferably, said IL-3 signaling inhibitor is an antibody or a fragment thereof capable of binding an antigen, an oligopeptide, or a small molecule inhibitor; wherein, more preferably, said IL-3 signaling inhibitor is an antibody or a fragment thereof capable of binding an antigen.

5. The IL-3 signaling inhibitor for use according to any of the foregoing claims, wherein the IL-3 signaling inhibitor is an inhibitor that targets IL-3, an inhibitor that targets interleukin-3 receptor (IL-3R), and/or an inhibitor that targets a complex formed by IL-3 and IL-3R;
wherein, preferably, said IL-3 signaling inhibitor is an anti-IL-3 antibody or an IL-3 binding fragment thereof, or an anti-IL-3R antibody or an IL-3R binding fragment thereof; more preferably an anti-IL-3 antibody or an IL-3 binding fragment thereof.

6. The IL-3 signaling inhibitor for use according to claim 5, wherein the IL-3 is human IL-3, the IL-3R is human IL-3R, and the complex formed by IL-3 and IL-3R is a complex formed by human IL-3 and IL-3R.

7. The IL-3 signaling inhibitor for use according to any of the foregoing claims, wherein said IL-3 signaling inhibitor is an anti-IL-3 antibody or an IL-3 binding fragment thereof that is capable of preventing IL-3 from binding to and/or activating the IL-3R upon binding of said anti-IL-3 antibody or said IL-3 binding fragment thereof to IL-3 in said patient; or
wherein said IL-3 signaling inhibitor is an anti-IL-3R antibody or an IL-3R binding fragment thereof that is capable of preventing IL-3 from binding to and/or activating the IL-3R upon binding of said anti-IL-3R antibody or an IL-3R binding fragment thereof to IL-3R in said patient.

8. The IL-3 signaling inhibitor for use according to any of the foregoing claims, wherein said IL-3 signaling inhibitor is an anti-IL-3 antibody or an IL-3 binding fragment thereof, wherein said anti-IL-3 antibody or IL-3 binding fragment thereof binds to the human IL-3 protein;
wherein, preferably, said anti-IL-3 antibody or IL-3 binding fragment thereof is not immunogenic in a human subject.

9. The IL-3 signaling inhibitor for use according to any of the foregoing claims, wherein either
a) said IL-3 signaling inhibitor is an anti-IL-3 antibody, wherein said anti-IL-3 antibody comprises an amino acid sequence which is at least 90%, preferably at least 95%, more preferably at least 98%, more preferably at least 99% identical to the amino acid sequence of an antibody selected from the group comprising:
- monoclonal anti-IL-3 antibody GRT002-H16L2
- monoclonal anti-IL-3 antibody Clone P8C11C8-6
- monoclonal anti-IL-3 antibody Clone 13.4.4;
preferably of an antibody from the group comprising:
- monoclonal anti-IL-3 antibody GRT002-H16L2
- monoclonal anti-IL-3 antibody Clone P8C11C8-6;
more preferably, of the monoclonal anti-IL-3 antibody GRT002-H16L2; or
b) said IL-3 signaling inhibitor is an IL-3 binding fragment of an anti-IL-3 antibody, wherein said IL-3 binding fragment comprises a fragment of an anti-IL-3 antibody which comprises an amino acid sequence which is at least 90%, preferably at least 95%, more preferably at least 98%, more preferably at least 99% identical to the amino acid sequence of an antibody selected from the group comprising:
- monoclonal anti-IL-3 antibody GRT002-H16L2
- monoclonal anti-IL-3 antibody Clone P8C11C8-6
- monoclonal anti-IL-3 antibody Clone 13.4.4;
preferably of an antibody from the group comprising:
- monoclonal anti-IL-3 antibody GRT002-H16L2
- monoclonal anti-IL-3 antibody Clone P8C11C8-6;
more preferably, of the monoclonal anti-IL-3 antibody GRT002-H16L2.

10. The IL-3 signaling inhibitor for use according to any of the foregoing claims, wherein said IL-3 signaling inhibitor is an anti-IL-3 antibody or an IL-3 binding fragment thereof, wherein said anti-IL-3 antibody or said IL-3 binding fragment thereof binds to an epitope within an amino acid sequence which is at least 90% identical, preferably at least 95 % identical, more preferably at least 98% identical, even more preferably at least 99% identical, optionally identical, to the mature human IL-3 amino acid sequence (SEQ ID NO: 1);
wherein, preferably, said anti-IL-3 antibody or said IL-3 binding fragment thereof binds to a portion of an IL-3 amino acid sequence which is at least 90% identical, preferably at least 95 % identical, more preferably at least 98% identical, even more preferably at least 99% identical, optionally identical, to residues 22-48 of the mature human IL-3 amino acid sequence (SEQ ID NO: 2).

11. The IL-3 signaling inhibitor for use according to any of the foregoing claims, wherein said IL-3 signaling inhibitor is a monoclonal, polyclonal, or chimeric antibody, or a combination thereof.

12. The IL-3 signaling inhibitor for use according to any of the foregoing claims, wherein the patient is characterized as a patient with a Long-COVID score of 1, 2, 3, or 4; preferably, a Long-COVID score of 2, 3, or 4; more preferably a Long-COVID score of 2 or 3; most preferably a Long-COVID score of 3.

13. A pharmaceutical composition comprising an IL-3 signaling inhibitor as defined in any of the foregoing claims, for use in a method of treatment of Long-COVID and/or Post-COVID syndrome after infection with a coronavirus, wherein, preferably, said composition further comprises at least one pharmaceutically acceptable carrier, diluent and/or excipient.

14. The IL-3 signaling inhibitor for use according to any of claims 1-12, or the pharmaceutical composition for use according to claim 13, wherein said IL-3 signaling inhibitor or said pharmaceutical composition is administered to a patient in need thereof, wherein, preferably, said patient is a human.

15. The IL-3 signaling inhibitor for use or the pharmaceutical composition for use according to claim 14, wherein said IL-3 signaling inhibitor or said pharmaceutical composition is administered in combination with one or more other therapies.
